Europäisches Patentamt

European Patent Office

Office européen des brevets

(11) Publication number: **0 451 932 A1**

## EUROPEAN PATENT APPLICATION

(21) Application number: **91250097.2**

(51) Int. Cl.⁵: **A61B 17/58**

(22) Date of filing: **10.04.91**

(30) Priority: **12.04.90 DE 4012506**

(43) Date of publication of application:
**16.10.91 Bulletin 91/42**

(84) Designated Contracting States:
**AT BE CH DE DK ES FR GB GR IT LI LU NL SE**

(71) Applicant: **MECRON MEDIZINISCHE PRODUKTE GMBH**
**Nunsdorfer Ring 23-29**
**W-1000 Berlin 48(DE)**

(72) Inventor: **Fischer, Hans-Joachim, Dr.-Ing.**
**Messmerstrasse 10**
**W-1000 Berlin 48(DE)**
Inventor: **Kranz, Curt, Dr.-Ing.**
**Kufsteiner Strasse 12**
**W-1000 Berlin 62(DE)**
Inventor: **Sauer, Günther, Dr.-Ing.**
**Fregestrasse 72**
**W-1000 Berlin 41(DE)**

(74) Representative: **Christiansen, Henning, Dipl.-Ing.**
**Patentanwalt Pacelliallee 43/45**
**W-1000 Berlin 33(DE)**

(54) **Bone screw.**

(57) Bone screw (1) formed of a material which is resorbable by the body comprising a recess (5) for the introduction of an insertion tool (8) whose cross-section is non-circular and suited for the transferral of torque, characterised in that the recess (5) for the introduction of the insertion tool (8) is situated in the head (4) of the bone screw (1) and that from the recess (5) there extends a through-going bore (6) for the insertion of a guide pin (9) to the tip (7) of the bone screw (1) and that the bore (6) comprises a round cross-section smaller than the cross-section of the recess (5).

Fig.1

The present invention relates to a bone screw and more particularly to a bone screw formed of a material that resorbs upon contact with body fluids comprising a recess for the introduction of an insertion tool whose cross-section is non-circular and suitable for the transferral of torque.

Bone screws formed of a resorbable plastic material, in particular polyactide or poly(L-lactide) are known and are used instead of non-resorbable bone screws made of stainless steel or titanium for orthopaedic fixation. An advantage of these bone screws is that they do not have to be removed from the body after the bone fracture has healed as the bone screw degrades and dissolves with time due to a resorption process.

It is disadvantageous that the plastic material used is appreciably less strong than the material of non-resorbable bone screws which can lead to the bone screws breaking when they are being screwed in as the torsional force exerted by the screwing instrument exceeds the breaking point load of the screw. The breaking point load is exceeded when the bone screw encounters unexpected resistance or jams in some other manner.

A known resorbable bone screw is described in DE-U 88 04 456 in which an insertion tool, whose cross-section is non-circular is inserted in a noncicurlar recess in order to acheive an uniform distribution of torsional forces in the shaft when the bone screws are screwed in.

It is disadvantageous that due to the relatively large recess a large amount of body fluids can get into the inside of the bone screw so that the bone screw is resorbed more quickly than the load-bearing ability of the surrounding bone increases. It is generally therefore necessary to provide this bone screw with an additional stopper which does not, however, improve the static or dynamic strength of the bone screw and in addition only forms a further alien body which must also be resorbed. Furthermore, the insertion of the stopper requires that an added hand movement is necessary when inserting the bone screw.

It is an object of the present invention to provide greater safety when handling the bone screw of the above-mentioned type and furthermore to enable a resorption to take place which is adapted to correspond with the growth of the bone without having to use an additional stopper.

The above and other objects are accomplished according to one aspect of the invention in that a recess for the introduction of an insertion tool is situated in the head of the bone screw and that from this recess there extends a through-going bore for the insertion of a guide pin as far as the tip of the bone screw and that the bore is of a round cross-section and that this cross-section is smaller than the cross-section of the recess.

The invention is based on the realization that a twisting or a jamming of the bone screw formed of a resorbable and therefore softer material is caused by an increased loading of the bone screw and which can lead to the bone screw breaking can be prevented by increasing the longitudinal rigidity of the bone screw whilst it is being screwed in. In order to increase the longitudinal rigidity of the bone screw no torque transferral is neccesary along the shaft, which in itself would lead to a further reduction in the strength of the shaft cross-section due to the related enlargement of the cross-section of the through-going bore. All that is required is a round bore and a guide pin which is inserted therein and which is dimensioned so as to correspond with the round bore to ensure that even with the shaft cross-section at a maximum a jamming of the bone screw, which enhances the chances of the bone screw twisting, is prevented. In addition a so-called "misdirection" of the bone screw, which can also, due to the change of direction, lead to a jamming and thus to a sudden increase in the screwing moment, can also be prevented by the round bore and the guide pin inserted therein.

The inventive embodiment is furthermore advantageous in that due to the provision of the smaller bore for the guide pin the bone screw is of a larger material cross-section which guarantees that the resorbable bone screw is stronger than the known bone screws when it is being screwed into the bone.

In addition there is another advantage, that due to the smaller bore it does not have to be closed over with an additional stopper as the small amount of body liquid which enters the bone screw does not accelerate the resorption process greatly. The bore, on the contrary, due to its uniform cross-section, even leads to the bone screw being evenly and uniformly degraded and absorbed.

It is also extremely advantageous with the bone screw according to the invention, when the sizes of the mean wall cross-sections in the shaft and head regions of the bone screw are essentially the same, so that a near overall uniformity of the wall thicknesses can be acheived so that an even and uniform resorption can be guaranteed if one presumes that all of the outer surface areas of the bone screw are degraded and dissolve at essentially the same rate.

The invention will be understood more fully, while still further objects and advantages will become apparent, in the following detailed description of a preferred embodiment of the invention illustrated in the accompanying drawings, in which:

Figure 1 is a cross-section of a bone screw according to the invention;

Figure 2 is a top elevational view of the bone screw of Figure 1; and

Figure 3 is a perspective view of the bone screw of Figure 1 with inserted insertion tool.

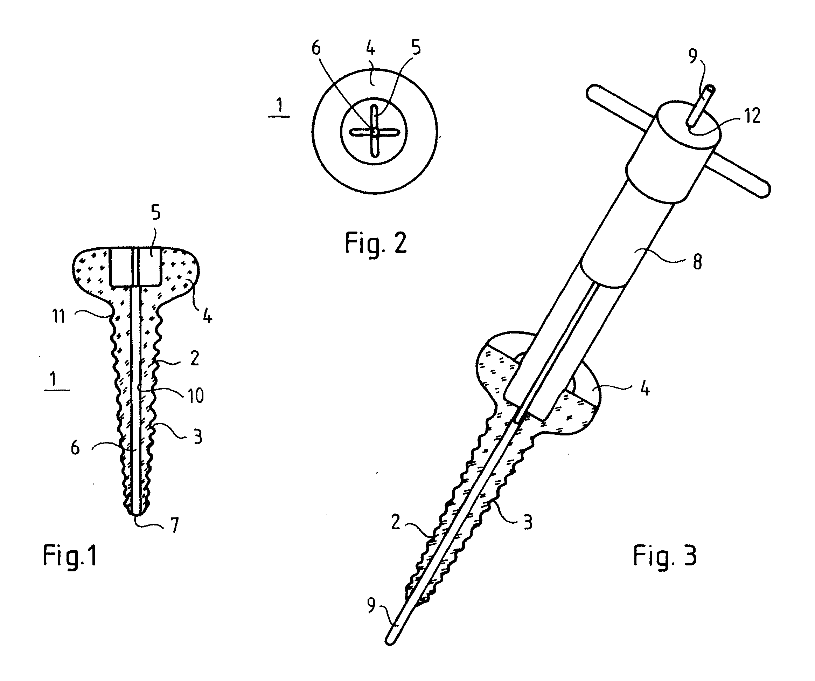

In the preferred embodiment of the invention in Figure 1 the bone screw 1 whose shaft 2 is threaded 3 is shown in cross-section. The head 4 of the bone screw 1 is rounded off in an outward direction.

The inner cross-section of the head 4 of the bone screw 1 comprises a recess 5, whose cross-section is non-circular in the head region only and is, in this embodiment, in the form of a cross. The recess 5 acts to receive an insertion tool 8, whose cross-section is also formed in the shape of a cross and is shaped complimentary to the recess 5 and is suitable for the transferral of torque.

Starting from the recess 5 there is a through-going round bore 6 to the tip 7 of the bone screw 1. In the shown embodiment the bore 6 has a diameter of approximately 1,5 mm. This bore 6 is used to receive a guide pin 9 with a round cross-section which is adapted to correspond with that of the bore 6 and which is smaller than the cross-section of the recess 5.

Body fluids enter and are in contact with the wall of the bore 6 of the bone screw 1 after it has been screwed in and the guide pin 9 has been removed from the bore 6. This surface area 10 dissolves and is resorbed in time if it is in contact with body fluids.

The bone screw 1 comprises mean wall thicknesses in the shaft- and head regions 2 and 4 which correspond approximately with one another. If one presumes, that all surface areas 10, which are in contact with body fluids are degraded at an even rate this embodiment ensures that the bone screw 1 as a unit is resorbed uniformly. Even if one surface area or a surface region 11 of the bone screw 1 is not dissolved by body fluids which can occur if the bore 6 is blocked unintentionally or if the body fluids are not in contact with the outer surface area of the bone screw 1 the exposed areas are still evenly resorbed.

Due to the cross-shape of the recess 5 in the head 4 of the bone screw 1 the penetration area for body fluids in this area is greater and the wall thickness which has to be penetrated corresponds to the wall thickness in the shaft area 2 of the bone screw 1 so that the overall rate of resorption is uniform. In particular, the shortest distance from a surface which is in contact with body fluids 10 to an opposite surface 11 which is not in contact with body fluids is essentially the same throughout the bone screw 1 or else is the equivalent of half the distance to another surface 10 which is in contact with body fluids, so that the cross-sections which have to be degraded are approximately of uniform thickness.

A plan view of the cross-shaped recess 5 in the head 4 of the bone screw 1 and the bore 6 which runs from the recess 5 is shown in Figure 2. The diameter of the bore 6 corresponds with that of the central area of the cross and is equivalent to the length of the diagonal of the square created by the two arms of the cross in the region in which they cross each other.

In Figure 3 a part of the insertion tool 8 is illustrated which comprises a cross-shaped cross-section adapted to correspond with the dimensions of the cross-shaped recess 5 of the bone screw 1 of Figure 1. Torque is transmitted by this insertion tool 8 during the insertion of the bone screw 1.

As can be seen from the Figure the insertion tool 8 comprises a through-going bore 12, through which the guide pin 9 can be inserted. The diameter of the guide pin 9 is adapted to correspond with the diameter of the bore 12 of the bone screw 1 so that it is a tight fit with essentially no clearance. In order to insert the bone screw 1 a guide bore with a smaller diameter is drilled in the bone screw 1 in the required direction. The guide pin 9 is then inserted into this guide bore and the bone screw 1 is then pushed over the guide pin 9 and is screwed in. Due to the guiding function of the pin 9 the resorbable bone screw 1 is guided in the required direction and is on the other hand saved from bending and twisting and the screw moment does not exceed the maximum allowable value.

It will be understood that the above description of the present invention is susceptible to various modifications, changes and adaptations, and the same are intended to be comprehended within the meaning and range of the equivalents of the appended claims.

## Claims

1. Bone screw formed of a material resorbable by the body and which comprises a recess for the introduction of an insertion tool whose cross-section is non-circular and is suited for the transferral of torque, characterised in that the recess (5) for the introduction of the insertion tool (8) is situated in the head (4) of the bone screw (1) and the bone screw (1) comprises a through-going bore (6) for the insertion of a guide pin (9) which extends from the recess (5) to the tip (7) of the bone screw (1) and whereby the through-going bore (6) comprises a round, cross-section smaller than the cross-section of the recess (5).

2. Bone screw according to claim 1, characterised in that the recess (5) is formed in the shape of a cross.

3. Bone screw according to claim 2, characterised in that the diameter of the through-going bore (6) is essentially equivalent to the length of the diagonal of the central area of said cross-shaped recess (5).

4. Bone screw according to any one of the preceding claims, characterised in that the mean size of the wall cross-section is essentially constant in the shaft (2) and head (4) region of the bone screw (1).

5. Bone screw according to any one of the preceding claims, characterised in that the shortest distance from a surface area (10) of the bone screw (1) which is in contact with body fluids to a surface area (11) of the bone screw (1) which is not in contact with body fluids is approximately the same or corresponds to half the distance to another surface area (10) which is also in contact with body fluids.

Fig. 2

Fig.1

Fig. 3

EP 0 451 932 A1

European
Patent Office

**EUROPEAN SEARCH
REPORT**

Application Number

**EP 91 25 0097**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | WO-A-8 909 030 (AESCULAP)<br>* Figure 7; page 9, lines 20-25 *<br>– – – | 1 | A 61 B 17/58 |
| A | EP-A-0 172 130 (MECRON)<br>* Figure 1; page 9, lines 14-19 *<br>– – – | 1 | |
| A | EP-A-0 260 222 (ILLI)<br>* Column 4, lines 29-32; figures 5,7 *<br>– – – – – | 1 | |
| | | | **TECHNICAL FIELDS SEARCHED (Int. Cl.5)**<br><br>A 61 B |

The present search report has been drawn up for all claims

| Place of search | Date of completion of search | Examiner |
|---|---|---|
| The Hague | 18 June 91 | BARTON S.A. |

CATEGORY OF CITED DOCUMENTS

X : particularly relevant if taken alone
Y : particularly relevant if combined with another
document of the same catagory
A : technological background
O : non-written disclosure
P : intermediate document
T : theory or principle underlying the invention

E : earlier patent document, but published on, or after
the filing date
D : document cited in the application
L : document cited for other reasons

&: member of the same patent family, corresponding
document